**Europäisches Patentamt**

**European Patent Office**

**Office européen des brevets**

(19)

(11) Veröffentlichungsnummer : **0 065 748**
**B1**

(12) # EUROPÄISCHE PATENTSCHRIFT

(45) Veröffentlichungstag der Patentschrift :
05.09.84

(51) Int. Cl.³ : **C 07 D501/18**

(21) Anmeldenummer : **82104368.4**

(22) Anmeldetag : **18.05.82**

(54) Verfahren zur Herstellung eines Cephemcarbonsäurederivats.

(30) Priorität : **22.05.81 CH 3362/81**

(43) Veröffentlichungstag der Anmeldung :
**01.12.82 Patentblatt 82/48**

(45) Bekanntmachung des Hinweises auf die Patenterteilung : **05.09.84 Patentblatt 84/36**

(84) Benannte Vertragsstaaten :
**AT BE CH DE FR GB IT LI NL**

(56) Entgegenhaltungen :
**EP-A- 0 000 005**
**DE-A- 2 804 896**

(73) Patentinhaber : **F. HOFFMANN-LA ROCHE & CO.**
**Aktiengesellschaft**
**CH-4002 Basel (CH)**

(72) Erfinder : **Hug, Rudolf Peter, Dr.**
**Im Hirshalm 48**
**CH-4125 Riehen (CH)**

(74) Vertreter : **Lederer, Franz, Dr. et al**
**Patentanwälte Dr. Franz Lederer Reiner F. Meyer**
**Lucile-Grahn-Strasse 22**
**D-8000 München 80 (DE)**

## Beschreibung

Die (7R, 8R)-7-Amino-[[(2,5-dihydro-6-hydroxy-2-methyl-5-oxo-as-triazin-3-yl)-thio]-methyl]-3-cephem-4-carbonsäure ist ein wertvolles Zwischenprodukt für die Herstellung von antibakteriell wirksamen Cephalosporin-derivaten, beispielsweise der (7R, 8R)-7-[2-(2-Amino-4-thiazolyl)-2-(Z-methoxyimino)-acetamido]-3-[[(2,5-dihydro-6-hydroxy-2-methyl-5-oxo-as-triazin-3-yl)-thio]-methyl]-3-cephem-4-carbonsäure. Diese Derivate lassen sich auf bekannte Weise aus dem genannten Zwischenprodukt durch Acylierung der 7-Aminogruppe mit entsprechenden Carbonsäuren oder reaktionsfähigen Carbonsäurederivaten erhalten.

Es ist bekannt, beispielsweise aus der deutschen Offenlegungsschrift 2 804 896, dass sich Verbindungen vom Typ des Eingangs genannten Zwischenprodukts, also (7R, 8R)-7-Amino-3-thiomethyl-3-cephem-4-carbonsäuren mit substituierter Thiogruppe dadurch herstellen lassen, dass man die (7R, 8R)-7-Amino-cephalosporansäure mit einer Thiolverbindung in Gegenwart von Bortrifluorid oder einer Komplexverbindung davon in einem polaren organischen Lösungsmittel umsetzt. Die Aufarbeitung aus der Reaktionslösung erfolgt bei dieser bekannten Methode durch Alkalisierung mit wässriger Base bis zu einem pH von etwa 3,5-4,0, wobei das Produkt in Form der freien Carbonsäure ausfällt, welche anschliessend abgetrennt, gewaschen und getrocknet wird.

Wird in diesem Verfahren als Thiolverbindung das 2,5-Dihydro-6-hydroxy-3-mercapto-2-methyl-5-oxo-as-triazin eingesetzt, erhält man bei der üblichen Aufarbeitung mit wässriger Base bei pH 2,5-4,0 das gewünschte Zwischenprodukt, nämlich die (7R, 8R)-7-Amino-3-[[(2,5-dihydro-6-hydroxy-2-methyl-5-oxo-as-triazin-3-yl)-thio]-methyl]-3-cephem-4-carbonsäure überraschenderweise nicht gänzlich in Form der freien Carbonsäure, sondern als Gemisch mit dem Monosalz (z. B. als Gemisch mit dem Monoammoniumsalz). Dieses Gemisch ist mit bestimmten Nachteilen verbunden, indem es mit einem beträchtlichen Anteil unidentifizierter Verunreinigungen (> 10 %) anfällt. Ferner sind in der Regel Monosalze der Carbonsäure für die anschliessende 7-Acylierung nicht oder nur schlecht geeignet, da die 7-Acylierung bevorzugt in wasserfreier Lösung, d. h. in Lösung in einem organischen Lösungsmittel, durchgeführt werden soll und zwar wegen der damit verbundenen Vorteile bezüglich der Stabilität der Reaktionsteilnehmer sowie der Ausbeuten und Reinheit des Reaktionsproduktes. Die für die 7-Acylierung gewünschte freie Carbonsäure ist im organischen Lösungsmittel zwar nicht an sich löslich, sie ist jedoch durch Silylierung oder Umsetzung mit einem Trialkylamin leicht in Lösung zu bringen. Die Monosalze sind (sofern sie nicht Trialkylammoniumsalze sind) jedoch weder im organischen Lösungsmittel löslich noch sind sie in besagter Weise in Lösung zu bringen ; sie sind deshalb, wie bereits gesagt, ungeeignete Ausgangsstoffe für die 7-Acylierung.

Es wurde nun gefunden, dass die (7R, 8R)-7-Amino-3-[[(2,5-dihydro-6-hydroxy-2-methyl-5-oxo-as-triazin-3-yl)-thio]-methyl]-3-cephem-4-carbonsäure gänzlich als die gewünschte, freie Carbonsäure erhalten wird, wenn man den pH-Wert auf etwa 1,4-2,0, insbesondere auf 1,6-1,8, einstellt. Es ist dabei überraschend, dass bei diesem pH-Wert die 7-Aminogruppe nicht als Säureadditionssalz vorliegt, in welcher Form sie für anschliessende Acylierungsreaktionen blockiert und damit nicht verwendbar wäre.

Durch die Ausfällung der (7R, 8R)-7-Amino-3-[[(2,5-dihydro-6-hydroxy-2-methyl-5-oxo-as-triazin-3-yl)-thiol]-methyl]-3-cephem-4-carbonsäure als freie Carbonsäure bei einem pH-Wert von etwa 1,4-2,0 erhält man nun nach Abtrennung, z. B. durch Filtration, sowie Waschen mit üblichen Lösungsmitteln und Trocknen, ein besonders reines, nämlich ein in der Regel etwa 98-100 %ig reines Produkt (entsprechende Reinheit des bei pH 3,5-4,0 ausgefällten Gemisches von Carbonsäure/Natriumsalz ist > 90 %), das im Gegensatz zum Ammoniumsalz in nicht-wässriger, organischer Phase mit Silylierungsmitteln, z. B. mit N,O-Bis-(trimethylsilyl)-acetamid, Trimethylsilylacetamid, Trimethylchlorsilan oder Dimethyldichlorsilan, oder mit einem Trialkylamin, z. B. Triäthylamin, in das entsprechende, lösliche Produkt umgewandelt werden kann. Letzteres kann anschliessend in der organischen Phase direkt mit dem Acylierungsmittel in hoher Ausbeute und Reinheit in entsprechende, antibakteriell wirksame Cephalosporinderivate übergeführt werden.

Das erfindungsgemässe Verfahren betrifft demgemäss die Herstellung von (7R, 8R)-7-Amino-3-[[(2,5-dihydro-6-hydroxy-2-methyl-5-oxo-as-triazin-3-yl)-thiol]-methyl]-3-cephem-4-carbonsäure durch Umsetzung von (7R, 8R)-7-Amino-cephalosporansäure mit 2,5-Dihydro-6-hydroxy-3-mercapto-2-methyl-5-oxo-as-triazin in Gegenwart von Bortrifluorid oder einer Komplexverbindung davon in einem polaren organischen Lösungsmittel und ist dadurch gekennzeichnet, dass man in wässriger Phase das Reaktionsprodukt in Form der freien Säure durch Zusatz einer Base bis zu einem pH-Wert von etwa 1,4-2,0 ausfällt, worauf die reine Säure abgetrennt werden kann.

Die Umsetzung der obigen Reaktionsteilnehmer erfolgt in bekannter Weise in Gegenwart von Bortrifluorid oder einer Komplexverbindung davon, z. B. in Gegenwart des Komplexes mit Diäthyläther, Essigsäure, Acetonitril usw. In der Regel wird das Bortrifluorid bzw. eine seiner Komplexverbindungen in molarem Ueberschuss, z. B. 2 bis 7 Mol pro Mol (7R, 8R)-7-Amino-cephalosporansäure, verwendet. Die Umsetzung erfolgt in wasserfreiem Medium, und zwar in einem polaren organischen Lösungsmittel, vorzugsweise in Acetonitril oder Eisessig, und die Reaktionstempertatur liegt, obwohl nicht kritisch, bevorzugt zwischen Zimmertemperatur und etwa 50 °C.

Nach vollendeter Reaktion wird das Reaktionsgemisch vorzugsweise zunächst mit Wasser auf einen Wassergehalt von etwa 15-30 Vol.-% verdünnt. Anschliessend wird der pH-Wert mit Hilfe einer geeigneten Base, z. B. mit Natron- oder Kalilauge, mit Ammoniak, auf einen pH-Wert von etwa 1,4-2,0, vorzugsweise 1,6-1,8, eingestellt, wobei das Reaktionsprodukt ausfällt. Der Wassergehalt soll bei der Ausfällung vorzugsweise innerhalb der Grenzen von etwa 15-30 Vol.-% bleiben. Diese Grenzen können z. B. dadurch eingehalten werden, dass man das benötigte Wasser zur hauptsache vor der Base zusetzt, die dann anschliessend in wasserfreier oder wässrigkonzentrierter Form zugegeben werden kann. Man kann auch zunächst nur wenig oder überhaupt kein Wasser zusetzen und anschliessend entsprechend verdünnte wässrige Base verwenden. Als praktische Mittellösung ist es zweckmässig, zunächst nur einen Teil des Wassers (z. B. etwa 15-25 Vol.-%) zuzusetzen und dann das Gemisch mit entsprechend verdünnter wässriger Base bis auf einen Wassergehalt von etwa 20-30 Vol.-% und einen pH-Wert von etwa 1,4-2,0 einzustellen.

Obwohl die Erfindung nicht auf die Anwendung eines Wassergehaltsbereiches von etwa 15-30 Vol.-% beschränkt ist, stellt dies eine vorteilhafte Ausführungsform des erfindungsgemässen Verfahrens dar. Bei geringerem Wassergehalt ist das Verfahren weniger vorteilhaft, weil dann die in der Reaktion als Nebenprodukt gebildete Borsäure auszufallen beginnt, wobei das Reaktionsprodukt verunreinigt wird. Bei höherem Wassergehalt erhält man ein schwerer filtrierbares Produkt, das sich beim Trocknen teilweise zersetzt.

Innerhalb der bevorzugten Grenzen von etwa 15-30 Vol.-% Wasser kann der ausgefällte Niederschlag in reiner Form leicht abgetrennt, z. B. durch Filtration, mit geeigneten Lösungsmitteln gewaschen, z. B. mit Wasser, Acetonitril/Wasser und/oder Aceton, und mit minimalem Zersetzungsverlust getrocknet werden. Man erhält so die (7R, 8R)-7-Amino-3-[[2,5-dihydro-6-hydroxy-2-methyl-5-oxo-as-triazin-3-yl)-thio]-methyl]-3-cephem-4-carbonsäure in besonders reiner (regelmässig 98-100 %) und stabiler Form.

Die folgenden Beispiele veranschaulichen die Erfindung, ohne sie zu beschränken.

Beispiel 1

30 g (7R, 8R)-7-Amino-cephalosporansäure und 17,5 g 2,5-Dihydro-6-hydroxy-3-mercapto-2-methyl-5-oxo-as-triazin werden in 350 ml Acetonitril aufgeschlämmt und unter Stickstoffbegasung auf 50 °C erwärmt. Sobald diese Temperatur erreicht ist, werden 73 g Bortrifluorid-ätherat zugegeben. Nach 30 Minuten wird auf 10 °C gekühlt und 116 ml Wasser zugegeben. Anschliessend wird der pH-Wert mit etwa 70 ml wässrigem 12 %igem Ammoniak auf 1,7 eingestellt, wobei das Produkt ausfällt. Nach 15-minütigem Rühren wird das Produkt abgenutscht, je zweimal mit 80 % Acetonitril/Wasser und Aceton gewaschen und getrocknet. Man erhält 33,0 g (80,6 %) (7R, 8R)-7-Amino-3-[[(2,5-dihydro-6-hydroxy-2-methyl-5-oxo-as-triazin-3-yl)-thio]-methyl]-3-cephem-4-carbonsäure vom Schmelzpunkt 195-198 °C (Zers.). Gehalt 98,0 % gemäss hochdruckflüssigchromatographischer Analyse.

Beispiel 2

30 g (7R, 8R)-7-Amino-cephalosporansäure und 17,5 g 2,5-Dihydro-6-hydroxy-3-mercapto-2-methyl-5-oxo-as-triazin werden in 235 ml Acetonitril aufgeschlämmt und unter Stickstoffbegasung auf 35 °C erwärmt. Sobald diese Temperatur erreicht ist, wird eine Lösung von 26,1 g Bortrifluorid in 135 ml Acetonitril zugegeben. Nach 30 Minuten wird auf 10 °C gekühlt und 128 ml Wasser zugegeben. Anschliessend wird der pH-Wert mit etwa 42 ml wässrigem 12 %igem Ammoniak auf 1,65 eingestellt, wobei das Produkt ausfällt. Nach 15-minütigem Rühren wird das Produkt abgenutscht, je zweimal mit 80 % Acetonitril/Wasser und Aceton gewaschen und getrocknet. Man erhält 33,5 g (81,7 %) (7R, 8R)-7-Amino-3-[[(2,5-dihydro-6-hydroxy-2-methyl-5-oxo-as-triazin-3-yl)-thio]-methyl]-3-cephem-4-carbonsäure vom Schmelzpunkt 201-203 °C (Zers.). Gehalt 99,5 % gemäss hochdruckflüssigchromatographischer Analyse.

**Ansprüche**

1. Verfahren zur Herstellung von (7R, 8R)-7-Amino-3-[[(2,5-dihydro-6-hydroxy-2-methyl-5-oxo-as-triazin-3-yl)-thio]-methyl]-3-cephem-4-carbonsäure durch Umsetzung von (7R, 8R)-7-Amino-cephalosporansäure mit 2,5-Dihydro-6-hydroxy-3-mercapto-2-methyl-5-oxo-as-triazin in Gegenwart von Bortrifluorid oder einer Komplexverbindung davon in einem polaren organischen Lösungsmittel, dadurch gekennzeichnet, dass man in wässriger Phase das Reaktionsprodukt in Form der Freien Säure durch Zusatz einer Base bis zu einem pH-Wert von etwa 1,4-2,0 ausfällt.

2. Verfahren nach Anspruch 1, dadurch gekennzeichnet, dass man als Base Ammoniak verwendet.

3. Verfahren nach Anspruch 1 oder 2, dadurch gekennzeichnet, dass man den pH-Wert auf 1,6-1,8 einstellt.

4. Verfahren nach einem der Ansprüche 1,3, dadurch gekennzeichnet, dass man die Ausfällung unter Beibehaltung eines Wassergehaltes von etwa 15-30 Vol.-% vornimmt.

5. Verfahren nach Anspruch 4, dadurch gekennzeichnet, dass man das Reaktionsgemisch vorgängig des Basenzusatzes mit Wasser auf einen Wassergehalt von etwa 15-25 Vol.-% verdünnt und anschliessend entsprechend verdünnte wässrige Base bis auf einen Wassergehalt von etwa 20-30 Vol.-% zusetzt.

## Claims

1. A process for the manufacture of (7R, 8R)-7-amino-3-[[(2,5-dihydro-6-hydroxy-2-methyl-5-oxo-as-triazin-3-yl)-thio]-methyl]-3-cephem-4-carboxylic acid by reacting (7R, 8R)-7-amino-cephalosporanic acid with 2,5-dihydro-6-hydroxy-3-mercapto-2-methyl-5-oxo-as-triazine in the presence of boron trifluoride or a complex compound thereof in a polar organic solvent, characterized by precipitating the reaction product in an aqueous phase in the form of the free acid by adding a base up to a pH-value of about 1.4-2.0.

2. A process according to claim 1, characterized in that ammonia is used as the base.

3. A process according to claim 1 or 2, characterized in that the pH-value is adjusted to 1.6-1.8.

4. A process according to any one of claims 1-3, characeerized in that the precipitation is carried out while maintaining a water content of about 15-30 vol.-%.

5. A process according to claim 4, characterized in that the raction mixture is diluted with water to a water content of about 15-25 vol.-% prior to the addition of the base and appropriately diluted aqueous base is subsequently added up to a water content of about 20-30 vol.-%.

## Revendications

1. Procédé de préparation d'acide (7R, 8R)-7-amino-3-[[(2,5-dihydro-6-hydroxy-2-méthyl-5-oxo-as-triazin-3-yl)-thio]-méthyl]-3-céphem-4-carboxylique par réaction d'acide (7R, 8R)-7-amino-céphalosporanique avec de la 2,5-dihydro-6-hydroxy-3-mercapto-2-méthyl-5-oxo-as-triazine en présence de trifluorure de bore ou d'un de ses composés complexes dans un solvant organique polaire, caractérisé en ce qu'on précipite le produit réactionnel en phase aqueuse sous la forme de l'acide libre par addition d'une base jusqu'à un pH d'environ 1,4-2,0.

2. Procédé selon la revendication 1, caractérisé en ce qu'on utilise comme base l'ammoniac.

3. Procédé selon l'une des revendications 1 ou 2, caractérisé en ce qu'on règle de pH à 1,6-1,8.

4. Procédé selon l'une des revendications 1-3, caractérisé en ce qu'on procéde à la précipitation en maintenant une teneur en eau d'environ 15-30 % en volume.

5. Procédé selon la revendication 4, caractérisé en ce qu'on dilue le mélange réactionnel avant d'ajouter la base avec de l'eau à une teneur en eau d'environ 15-20 % en volume puis en ce qu'on ajoute une base aqueuse diluée de façon correspondante jusqu'à une teneur en eau d'environ 20-30 % en volume.